# EUROPEAN PATENT APPLICATION

(11) **EP 3 061 423 A2**
(43) Date of publication of application: **31.08.2016**
(21) Application number: 16156740.9
(22) Date of filing: 22.02.2016
(51) Int. Cl.: A61F 2/42, A61B 17/29

(54) **DEVICE FOR TREATING FLAT FEET**

(30) Priority: 24.02.2015 IT PD20150046
(71) Applicant: Bidoia s.a.s. di Gianfranco Bidoia E C., 35010 Vigonza (Padova) (IT)
(72) Inventor: BIDOIA, Gianfranco, 35142 Padova (IT); CAVALIERI FOSCHINI, Andrea, 44029 Porto Garibaldi FE (IT)
(74) Representative: Modiano, Micaela Nadia

(57) **Abstract**

A device (10) for treating flat feet, comprising an expandable block (11) with an axial seat (12) for an expander insert (13) adapted to be inserted in the axial seat (12), the expandable block (11) comprising two opposite end parts (14, 15) and a central part (16), the two opposite end parts (14, 15) being configured to be deformed so as to increase their own radial space occupation with respect to the central part (16) when the expander insert (13) is arranged in the axial seat (12).

## Description

The present invention relates to a device for treating flat feet.

Currently flat feet is one of the most frequent orthopedic deformities in infancy and is characterized morphologically by a reduction of the plantar arch, by a valgoid condition of the hindfoot and functionally by a state of prevalent or persistent pronation during all phases of walking.

In order to try to solve the symptoms of discomfort, if not pain, linked to this defect, and prevent the associated pathologies, correction of the excessive pronation of the subastragalar joint during growth is usually recommended, so as to facilitate its remodeling and reestablish the normal anatomical relationships between the astragalus and the calcaneum.

At an age between four and eight, in the presence of a functional flatfoot, a conservative treatment is indicated by using an orthesis that stabilizes the hindfoot in a neutral position; this orthesis is constituted by a rigid shell to be worn of course at the arch of the foot.

Conservative treatment is rarely effective beyond eight years of age; in these cases surgical treatment of arthrorisis of the subastragalar joint is indicated.

This surgical treatment limits the articulation of the subastragalar joint by inserting an endorthesis which, by realigning the astragalus and the calcaneum, produces a partial and controlled mechanical braking of the joint, contrasts excessive pronation and allows the foot to develop correctly during growth.

A first type of endorthesis currently known is constituted by a cylindrical or slightly conical screw made of titanium (of medical grade), which, inserted appropriately in the bone structure of the foot, subjects it to a small helical rotation that is adapted to cause and obtain the appropriate curvature of the sole so as to eliminate or at least reduce flat foot.

This first type of endorthesis with a simple screw, despite its robustness and apparent simplicity, has proven limitations such as rigidity, poor adaptability and the concrete possibility of exit from the bones of the structure in which it is inserted.

In the search for the materials deemed most suitable to obviate the exit motion of such first type of endorthesis, so-called bioresorbable materials, such as for example polylactic acid or poly(lactic acid) and the like, have also been used in an attempt to provide a screw which, once the physiological absorption process has begun, does not undergo significant displacements.

However, the results have turned out to be below expectations.

In order to obviate the limitations of this first type of endorthesis, a second type of endorthesis with variable space occupation has been developed which are easy to insert in the location for which they are intended and are deformable after insertion, so as to obtain a radially expanded shape adapted for the purpose of keeping the endorthesis stably in position for the time need to achieve the sought result.

This second type of endorthesis has the appearance, for example, of an expansion device, such as the expansion screw of Prof. Giannini, which comprises an expandable block with an axial seat for an expander insert adapted to be inserted in such axial seat; such expandable block comprises two opposite end parts, at least one of which maintains substantially unchanged radial dimensions, and a substantially final part, which expands radially due to the axial expansion of such block, so as to assume an approximately frustum-like shape, like a typical Fischer expansion plug.

The expanded final part of the block is intended to apply pressure to the surrounding bone surfaces so as to influence the internal structure between the bones in which it has been inserted, so much as to space apart, with a movement that becomes "helical", the bone parts involved in the flat foot problem, until the necessary arc-like shape of the sole of the foot is obtained or approximated as much as possible.

The expandable block is made of a special plastic material, an ultrahigh density biocompatible polyethylene (UHMWPE), which has an excellent degree of mechanical, elastic and structural strength.

This second type of endorthesis, though being a step forward in the solution to the problem, also has limitations, which are due to a large extent to the final geometrical shape obtained with expansion, so that indeed due to this approximately frustum-like shape tends to migrate toward the inside of the bone structure of the foot, partly thwarting, often in rather short times, the benefit obtained with bone displacement.

A further consequent disadvantage of this second type of endorthesis is constituted by the fact that the displacement of the block frees small spaces in which such block can move slightly; this causes a defensive reaction of the surrounding tissues, which tend to interact in order to eliminate the foreign item, modifying the very structure of the plastic material, which becomes similar to a rubber and no longer achieves effectively the necessary expansion.

A second embodiment of this second type of endorthesis has an expandable block that expands radially on the external part with respect to a direction and orientation of insertion in the foot of the patient.

Though achieving the desired helical rotation of the bone bodies between which it is interposed, this endorthesis, also, has limitations:
- this endorthesis in any case obtains a "barrel-like" shape, with a larger diameter toward the outside, and even if it is provided with extraction-preventing protrusions these protrusions are not sufficient to eliminate the problem of the migration of the endorthesis toward the outside or inside of the foot;
- the titanium expander insert on the one hand increases the solidity of the mechanical assembly but on the other hand, indeed due to the strength of such material, often does not allow to obtain a sufficient expansion of the system.

Furthermore, the relationship between the plastic material and titanium is not easy to solve and the two elements are always subject to mutual axial displacements, with the consequent risk of exit and separation of the internal one from the external one.

The aim of the present invention is to provide a device for treating flat feet that is capable of obviating the cited limitations of endortheses of the known type.

Within this aim, an object of the invention is to provide a device the displacements of which with respect to a preset position in the foot of a patient are limited or absent.

Another object of the invention is to provide a device that can be inserted easily in the foot of a patient and is easy equally extractable with per se known operating methods.

A further object of the invention is to provide a device that does not free spaces in which said block can move undesirably, causing a defensive reaction of the surrounding tissues.

Another object of the invention is to provide a device the components of which are stably coupled to each other without the risk of separation.

Another object of the invention is to provide a tool for using a device according to the same invention.

This aim, as well as these and other objects that will become better apparent hereinafter, are achieved by a device for treating flat feet, comprising an expandable block with an axial seat for an expander insert adapted to be inserted in said axial seat, said device being characterized in that said expandable block comprises two opposite end parts and a central part, said two opposite end parts being configured to be deformed so as to increase their own radial space occupation with respect to said central part when said expander insert is arranged in said axial seat.

Further characteristics and advantages of the invention will become better apparent from the description of a preferred but not exclusive embodiment of the device according to the invention, illustrated by way of nonlimiting example in the accompanying drawings, wherein:
Figure 1 is an exploded perspective view of the device according to the invention;
Figure 2 is an exploded sectional side view of the device according to the invention;
Figure 3 is the same view of Figure 2 in the assembled condition;
Figure 4 is a top plan view of a component of the device according to the invention in a first configuration for use;
Figure 5 is a bottom plan view of the component of Figure 4;
Figure 6 is a side view of the component of Figures 4 and 5, taken along the sectional line VI-VI of Figure 5;

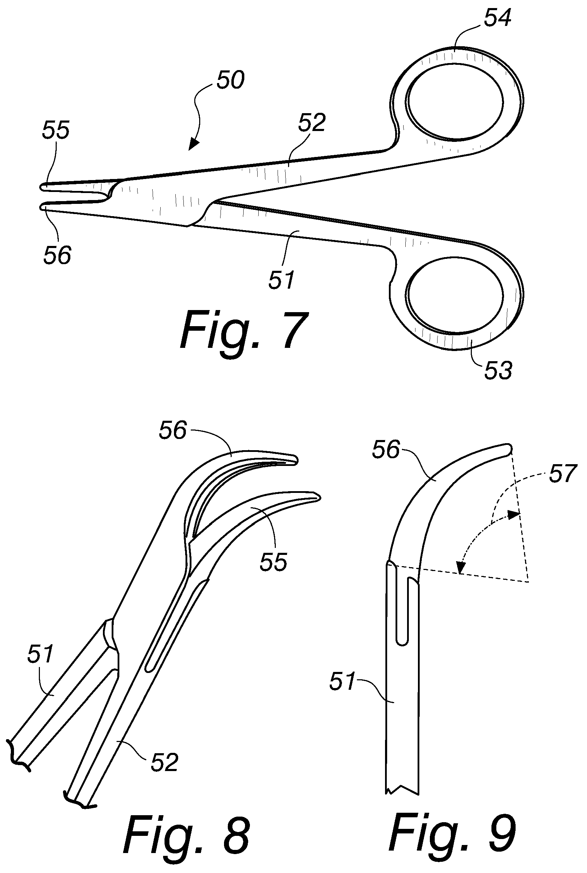

Figure 7 is a plan view of a tool according to the invention;
Figure 8 is a perspective view of a detail of the tool of Figure 7;
Figure 9 is a side view of a detail of the tool of Figures 7 and 8.

With reference to the figures, a device for treating flat feet according to the invention is designated generally by the reference numeral 10.

The device 10 comprises an expandable block 11, with an axial seat 12 for an expander insert 13 that is adapted to be inserted in the axial seat 12.

The expandable block 11 comprises two opposite end parts, a first leading end part 14 and a second trailing end part 15, and a central part 16.

The two opposite end parts 14 and 15 are configured to be deformed so as to increase their radial space occupation with respect to the central part 16 when the expander insert 13 is arranged in the axial seat 12.

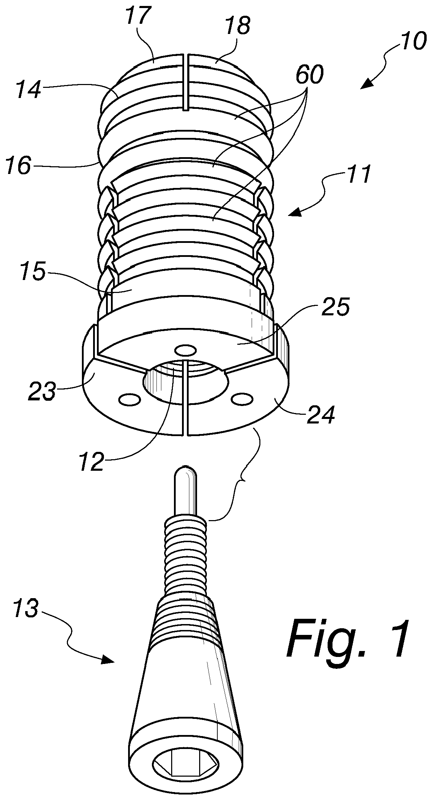

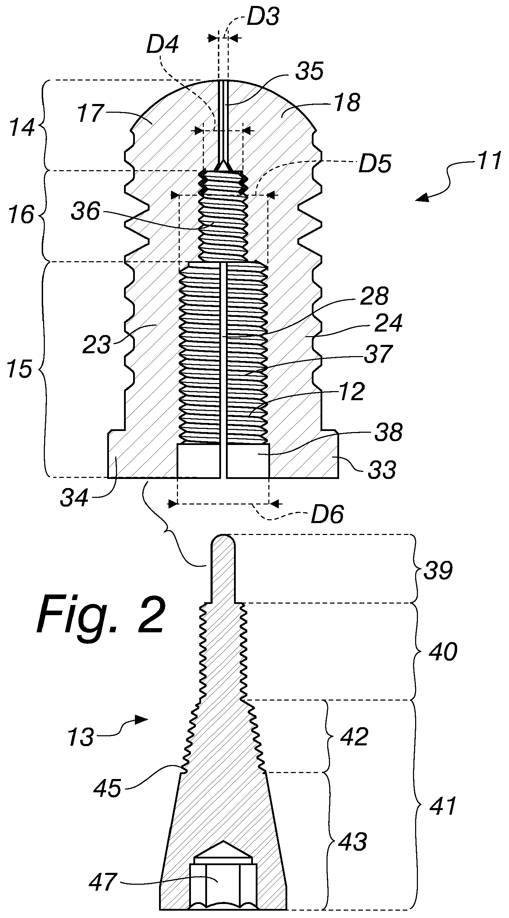

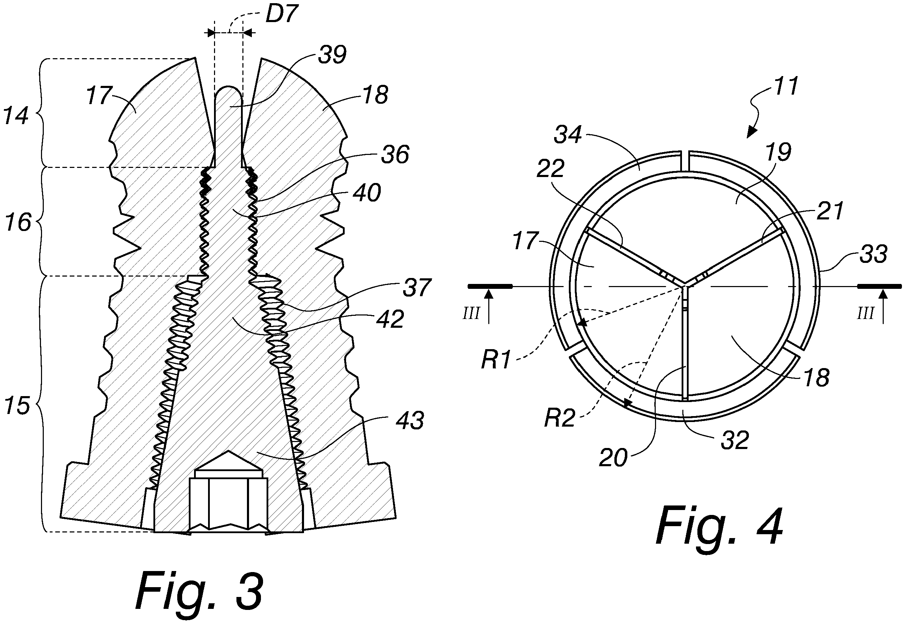

The expandable block 11 is therefore preset to assume two active configurations, a first insertion configuration, shown in Figures 1, 2, 4, 5 and 6, and a second open and active configuration, as shown in Figure 3.

The second configuration occurs when the expander insert 13 is inserted in the axial seat 12.

The end parts 14 and 15 are extended in one piece with the central part 16.

The one piece body that defines the expandable block 11 is preferably made of plastic material, such as for example but not exclusively UHMWPE polyethylene.

The expander insert 13 is constituted by an element made of a metallic material, for example titanium.

The expandable block 11 has a substantially cylindrical shape, with a rounded point at the first end part 14 in order to facilitate its insertion during the setup of the device 10.

In particular, the first leading end part 14 of the expandable block 11 comprises at least two sectors that can be divaricated, for example and preferably three head sectors 17, 18 and 19 that can be divaricated and are clearly visible in Figure 4, each having a cross-section in plan view that is shaped like a circular sector and being separated in pairs by corresponding radial separation interspaces 20, 21 and 22.

The three sectors 17, 18 and 19 that can be divaricated are mutually identical, each with a cross-section in plan view that has a center angle of 120°.

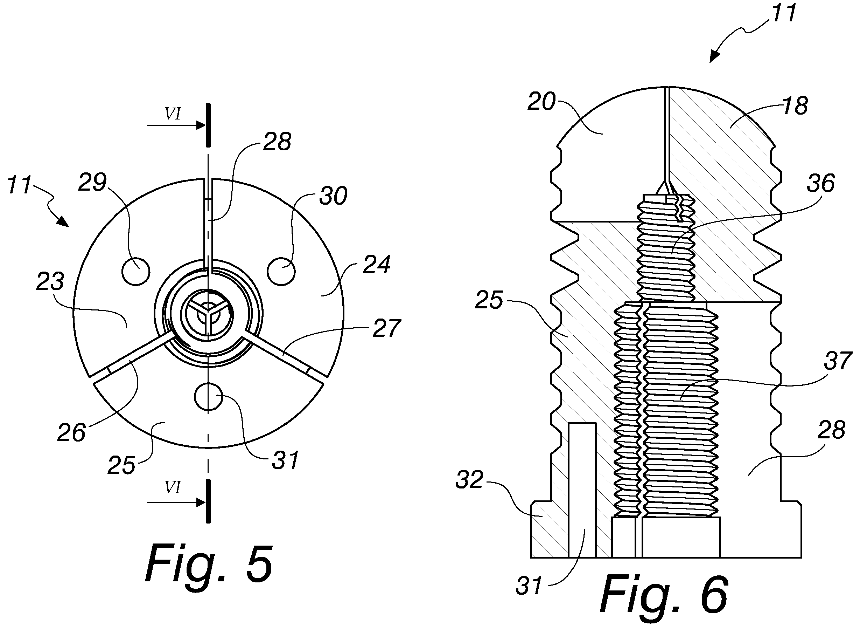

Likewise, the second trailing end part 15 of the expandable block 11 comprises at least two sectors that can be divaricated, for example and preferably three trailing sectors 23, 24 and 25 that can be divaricated and are clearly visible in Figure 5, each having a cross-section in plan view shaped like a circular sector and being separated in pairs by corresponding separation interspaces 26, 27 and 28.

The three trailing sectors 23, 24 and 25 that can be divaricated are mutually identical, each with a cross-section in plan view that has a center angle of 120°.

The three trailing sectors 23, 24 and 25 that can be divaricated are each provided with an actuation hole 29, 30 and 31 that is preset to accommodate a corresponding actuation point of a tool that is described hereinafter.

The three trailing sectors 23, 24 and 25 that can be divaricated are each provided at the end with an external perimetric portion 32, 33 and 34 respectively, which is clearly visible in Figure 4 and is extended radially with a radius R2 that is greater than the external radius R1 of the substantially cylindrical contour of the expandable block 11 in its first insertion configuration.

The external perimetric portions 32, 33 and 34 define an abutment in an axial direction in order to facilitate the placement of the expandable block 11 upon its insertion in the foot of the patient, being adapted to contrast any uncontrolled penetration motion of the expandable block 11 in its first insertion configuration.

The axial seat 12 for the expander insert 13 has, when the expandable block 11 is in the first insertion configuration:
- a through opening 35 at the first end part 14 of the expandable block 11, which is connected to the radial interspaces 20, 21 and 22 and has, in cross-section, a first reference diameter D3;
- a first internally threaded portion 36 at the central part 16 of the expandable block 11, which has, in cross-section, a reference diameter D4 that is greater than the reference diameter D3 of the through opening 35;
- a second internally threaded portion 37 at the second end part 15, which has, in cross-section, a reference diameter D5 that is greater than the reference diameter D4 of the first threaded portion 36; the second threaded portion 37 is divided between the sectors 23, 24 and 25 that can be divaricated and compose the second end part 15;
- an entry portion 38, which has a smooth lateral surface, with a reference diameter D6 that is greater than the reference diameter D5 of the second threaded portion 37.

The expander insert 13 comprises:
- a spike 39, which has a diameter D7 that is greater than the diameter D3 of the through opening 35 and is preset to divaricate the leading sectors 17, 18 and 19 that can be divaricated, as clearly visible in Figure 3,
- a cylindrical portion 40, which it is threaded complementarily externally with respect to the first internally threaded portion 36 and is therefore adapted to screw into it,
- a frustum-shaped portion 41 for the divarication of the trailing sectors 23, 24 and 25 that can be divaricated.

The frustum-shaped portion 41 comprises a threaded frustum-shaped portion 42 and a smooth frustum-shaped portion 43.

The threaded frustum-shaped portion 42 is contiguous to the cylindrical portion 40.

The threaded frustum shaped portion 42 has an external thread that has the same pitch as the thread of the cylindrical portion 40.

Correspondingly, the internally threaded portions 36 and 37 of the expandable block 11 have the same pitch.

In this constructive example, the last thread 45 of the threaded frustum-shaped portion 42 has such a diameter as to grip between the threads of the second internally threaded portion 37 of the second part 15 of the expandable block 11.

The invention also relates to a tool 50 for using the device 10.

The tool 50 is constituted by a clamp with two symmetrical arms 51 and 52 with grip portions 53 and 54 that are mutually pivoted and each of which is provided with a terminal with a point 55 and 56.

Each point 55 and 56 is contoured so that it can enter at least partially the actuation holes 29, 30 and 31 provided in the expandable block 11.

The points 55 and 56 are curved, with a circular arc-like profile with a center angle 57 of approximately 120°.

The tool 50 allows to prevent the rotation of the expandable block 11 while the expander insert 13 is screwed inside it, or allows to rotate the expandable block 11 for a better placement thereof in the insertion seat or for a movement thereof during extraction of the expandable block 11 from the foot of the patient.

The tool, by way of the pivoted arms 51 and 52, in fact allows to modify the distance between the points 55 and 56 to adapt to the actual distance, during the handling of the expandable block 11, of two of the actuation holes 29, 30 and 31.

Use of the device 10 according to the invention is as follows.

The expandable block 11 is inserted in the foot of the patient and its position is corrected, if necessary, with the tool with curved points 50.

The expander insert 13 is inserted in the axial seat 12 of the expandable block 11 and when the threaded frustum-shaped portion 42 encounters the thread of the second internally threaded portion 37 the expander insert 13 begins to be screwed in.

The expander insert 13 is provided with a recessed hexagon 47 for an Allen-type tool for screwing it in.

Simultaneously, if necessary, with the tool 50 with curved points the expandable block 11 is prevented from rotating during the screwing of the expander insert 13.

As screwing proceeds, the smooth frustum-shaped portion 43 divaricates the sectors 23, 24 and 25 that can be divaricated of the second end part 15, while the rest of the expandable block 11 is retained in its insertion configuration by the bones and by the surrounding tissues.

At a certain point the thread of the cylindrical portion 40 of the expander insert 13 grips the first internally threaded portion 36 at the central part 16, which does not expand, of the expandable block; as screwing in proceeds, the spike 39 advances in the through opening 35 and divaricates the sectors 17, 18 and 19 that can be divaricated of the first end part 14.

Once screwing in has ended, as shown in Figure 3, the two end parts 14 and 15 have an outside diameter that is greater than the outside diameter of the central part 16.

Advantageously, the expandable block 11 is provided externally with grip protrusions 60, which are adapted to improve the grip of the expandable block 11 on the tissues and the bone parts between which it is inserted.

The grip protrusions 60 are extended along the entire length of the expandable block 11 and can be constituted by circular ribs that surround the body of such block, or helical ones.

In practice it has been found that the invention achieves the intended aim and objects.

In particular, by means of the invention a device for treating flat feet has been perfected the displacements of which with respect to a preset position in the foot of a patient are limited or absent; this occurs by way of the expandable block, which can assume a first insertion configuration having a substantially cylindrical geometry, adapted for easy insertion in the established surgical site, and a second open active configuration, with the end parts divaricated with respect to the central part having a constant diameter.

Thanks to the deformable end parts, the expandable block assumes a shape that is similar to an hourglass, albeit not mirror-symmetrical and albeit, but not limitatively, with different dimensions.

Furthermore, the invention provides a device that can be inserted easily in the foot of a patient and can be extracted equally easily with per se known operating methods.

Moreover, the invention provides a device that does not free spaces in which such block can move undesirably, causing a defensive reaction of the surrounding tissues, since the contour with the two expanded end parts makes it impossible for the expandable block both to migrate toward the inside of the foot and to exit undesirably.

Furthermore, the invention provides a device the components of which are stably coupled to each other without the risk of separation thanks to the threaded coupling in the central part of the expandable block and of the expander insert, the extraction of which is highly unlikely.

Moreover, the invention provides a tool for using such a device for treating flat feet that is capable of blocking any unwanted rotation of the expandable block during insertion of the expander insert, allowing the optimum and complete insertion of the expander insert in the expandable block.

Moreover, the device according to the invention is reversible, i.e., it can be extracted/removed easily, both during surgical application (where and if necessary) and in a subsequent step, as required, even after considerable time, without particular negative consequences.

The invention thus conceived is susceptible of numerous modifications and variations, all of which are within the scope of the appended claims; all the details may further be replaced with other technically equivalent elements.

In practice, the materials used, so long as they are compatible with the specific use, as well as the contingent shapes and dimensions, may be any according to requirements and to the state of the art.

The disclosures in Italian Patent Application No. PD2015A000046 (102015902333198) from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device (10) for treating flat feet, comprising an expandable block (11) with an axial seat (12) for an expander insert (13) adapted to be inserted in said axial seat (12), said device being **characterized in that** said expandable block (11) comprises two opposite end parts (14, 15) and a central part (16), said two opposite end parts (14, 15) being configured to be deformed so as to increase their own radial space occupation with respect to said central part (16) when said expander insert (13) is arranged in said axial seat (12).

2. The device according to claim 1, **characterized in that** said end parts (14, 15) are extended in one piece with the central part (16).

3. The device according to one or more of the preceding claims, **characterized in that** said expandable block (11) has a substantially cylindrical shape, with a rounded tip at a first end part (14) in order to facilitate its insertion during the setup of the device (10).

4. The device according to one or more of the preceding claims, **characterized in that** said first leading end part (14) of the expandable block (11) comprises at least two head sectors (17, 18, 19) that can be divaricated and are separated by corresponding radial separation interspaces (20, 21, 22).

5. The device according to one or more of the preceding claims, **characterized in that** a second trailing end part (15) of the expandable block (11) comprises at last two trailing sectors (23, 24, 25) that can be divaricated and are separated in pairs by corresponding separation interspaces (26, 27, 28).

6. The device according to claim 5, **characterized in that** said trailing sectors (23, 24, 25) that can be divaricated are each provided with an actuation hole (29, 30, 31).

7. The device according to one or more of the preceding claims, **characterized in that** said axial seat (12) for the expander insert (13) has, when the expandable block (11) is in a first insertion configuration:
- a through opening (35) at the first end part (14) of the expandable block (11), which is connected to the radial interspaces (20, 21, 22) and has, in cross-section, a first reference diameter (D3);
- a first internally threaded portion (36) at the central part (16) of the expandable block (11), which has, in cross-section, a reference diameter (D4) that is greater than the reference diameter (D3) of the through opening (35);
- a second internally threaded portion (37) at the second end part (15), which has, in cross-section, a reference diameter (D5) that is greater than the reference diameter (D4) of the first threaded portion (36).

8. The device according to one or more of the preceding claims, **characterized in that** said expander insert (13) comprises:
- a spike (39), whose diameter (D7) is larger than the diameter (D3) of the through opening (35), preset to divaricate the head sectors (17, 18, 19) that can be divaricated;
- a cylindrical portion (40), which is threaded complementarily externally with respect to the first internally threaded portion (36) and is therefore adapted to screw into it,
- a frustum-shaped portion (41) for divarication of the trailing sectors (23, 24, 25) that can be divaricated.

9. The device according to one or more of the preceding claims, **characterized in that** said frustum-shaped portion (41) comprises a threaded frustum-shaped portion (42) and a smooth frustum-shaped portion (43).

10. A tool (50) for using a device (10) according to one or more of the preceding claims, **characterized in that** it comprises a clamp with two symmetrical arms (51, 52), with grip portions (53, 54) which are pivoted to each other, each provided with a terminal provided with a point (55, 56), each point (55, 56) being shaped so that it can enter at least partially in the actuation holes (29, 30, 31) that are present in the expandable block (11).
